# EUROPEAN PATENT SPECIFICATION

(11) **EP 1 371 736 B1**
(45) Date of publication and mention of the grant of the patent: **16.08.2006**
(21) Application number: 02386008.3
(22) Date of filing: 04.06.2002
(51) Int. Cl.: C12Q 1/70

(54) **Highly sensitive nested PCR detection scheme for efficient multiplex detection of members of Vitivirus, Foveavirus and Closterovirus genus in grapevine**
Hochsensitiver Nachweis mittels geschachtelter PCR zum effizienten multiplex Nachweis von Mitgliedern des Vitivirus, Foveavirus und Closteovirus Genus in Wein
Détection très sensible par PCR nichée pour la détection multiplexe efficace de membres du genre Vitivirus, Foveavirus et Closteovirus dans le vin

(43) Date of publication of application: 17.12.2003
(73) Proprietor: Vitro Hellas S.A., 53900 Alexandria (GR)
(72) Inventor: Dovas, Crisostomos, 54634 Thessaloniki (GR)

(56) References cited:
- SALDARELLI PASQUALE ET AL: "Use of degenerate primers in a RT-PCR assay for the identification and analysis of some filamentous viruses, with special reference to clostero- and vitiviruses of the grapevine." EUROPEAN JOURNAL OF PLANT PATHOLOGY, vol. 104, no. 9, December 1998 (1998-12), pages 945-950, XP008015613 ISSN: 0929-1873
- SEFC KRISTINA M ET AL: "Partial sequence identification of grapevine-leafroll-associated virus-1 and development of a highly sensitive IC-RT-PCR detection method." JOURNAL OF VIROLOGICAL METHODS, vol. 86, no. 1, April 2000 (2000-04), pages 101-106, XP002239073 ISSN: 0166-0934
- ROUTH GEOFFREY ET AL: "Use of degenerate partial sequencing and RT-PCR-based assays of grapevine leafroll-associated viruses 4 and 5." PHYTOPATHOLOGY, vol. 88, no. 11, November 1998 (1998-11), pages 1238-1243, XP000979592 ISSN: 0031-949X
- NASSUTH ANNETTE ET AL: "Improved RNA extraction and one-tube RT-PCR assay for simultaneous detection of control plant RNA plus several viruses in plant extracts." JOURNAL OF VIROLOGICAL METHODS, vol. 90, no. 1, October 2000 (2000-10), pages 37-49, XP002239074 ISSN: 0166-0934
- MACKENZIE D J ET AL: "IMPROVED RNA EXTRACTION FROM WOODY PLANTS FOR THE DETECTION OF VIRAL PATHOGENS BY REVERSE TRANSCRIPTION-POLYMERASE CHAIN REACTION" PLANT DISEASE, AMERICAN PHYTOPATHOLOGICAL SOCIETY, ST. PAUL, MN, US, vol. 81, no. 2, 1997, pages 222-226, XP001100054 ISSN: 0191-2917
- NOLASCO G ET AL: "Large scale evaluation of primers for diagnosis of rupestris stem pitting associated virus-1." EUROPEAN JOURNAL OF PLANT PATHOLOGY, vol. 106, no. 4, May 2000 (2000-05), pages 311-318, XP008015614 ISSN: 0929-1873
- LA NOTTE P ET AL: "A spot-PCR technique for the detection of phloem-limited grapevine viruses." JOURNAL OF VIROLOGICAL METHODS, vol. 66, no. 1, 1997, pages 103-108, XP000981995 ISSN: 0166-0934
- JAMES DELANO: "A simple and reliable protocol for the detection of apple stem grooving virus by RT-PCR and in a multiplex PCR assay." JOURNAL OF VIROLOGICAL METHODS, vol. 83, no. 1-2, December 1999 (1999-12), pages 1-9, XP002239076 ISSN: 0166-0934
- HATAYA T ET AL: "Molecular characterization of Hop latent virus and phylogenetic relationships among viruses closely related to carlaviruses." ARCHIVES OF VIROLOGY. AUSTRIA 2000, vol. 145, no. 12, 2000, pages 2503-2524, XP002239077 ISSN: 0304-8608
- ZHANG YUN-PING ET AL: "A strategy for rapid cDNA cloning from double-stranded RNA templates isolated from plants infected with RNA viruses by using Taq DNA polymerase." JOURNAL OF VIROLOGICAL METHODS, vol. 84, no. 1, January 2000 (2000-01), pages 59-63, XP002239075 ISSN: 0166-0934
- EHLERS BERNHARD ET AL: "Detection of new DNA polymerase genes of known and potentially novel herpesviruses by PCR with degenerate and deoxyinosine-substituted primers." VIRUS GENES, vol. 18, no. 3, 1999, pages 211-220, XP008015647 ISSN: 0920-8569
- DOVAS C I ET AL: "A spot nested RT-PCR method for the simultaneous detection of members of the Vitivirus and Foveavirus genera in grapevine." JOURNAL OF VIROLOGICAL METHODS, vol. 107, no. 1, 20 January 2003 (2003-01-20), pages 99-106, XP002239078 ISSN: 0166-0934

## Description

### FIELD OF THE INVENTION

The present invention relates to the use polymerase chain reaction (PCR) methods for detection of viruses, more particularly to degenerate deoxyinosine (dI)-substituted PCR primers and optimised nested PCR methods for simultaneously detecting *Vitivirus, Foveavirus,* and *Closterovirus* species, in grapevine.

### BACKGROUND OF THE INVENTION

Several important grapevine virus and virus-like diseases are transmitted and spread through the use of infected vegetatively propagated material. The major virus diseases of grapevine are: (1) the grapevine degeneration caused by *Grapevine fanleaf virus,* other European nepoviruses, and American nepoviruses, (2) the leafroll complex, and (3) the rugose wood complex (Martelli, ed., Graft Transmissible Diseases of Grapevines, Handbook for Detection and Diagnosis, FAO, UN, Rome, Italy 1993). The grapevine leafroll complex and the rugose wood complex are the most widely distributed throughout the world.

Leafroll is a serious virus disease of grapes. It affects almost all cultivated and rootstock varieties of Vitis, and it is of economical importance causing yield losses and reduction of sugar content. Annual yield losses of up to 20% have been estimated (Goheen, 1988, In: R. C. Pearson and A. C. Goheen (eds.), Compendium of Grape Diseases. American Phytopathological Society Press, St. Paul, Minnesota, USA, 53). Grapevine leafroll associated viruses (GLRaV) are a group of viruses that collectively or individually are associated with leafroll disease in grapevines. So far eight serologically distinct viruses *(Grapevine leafroll-associated virus-1, -2, -3, -4, -5, -6, -7,* and *-8* (GLRaV -1,-2,-3,-5,-6,-7)) have been found to be associated with the disease.

Rugose wood (RW) of grapevine is a complex of up to four distinct diseases, which occurs worldwide and is characterized by the development of pits and grooves on the woody cylinder. It is of great economic importance by causing severe reduction of growth and yield of affected grapevine plants (Garau et al., 1985, Phytopathologia Mediterranea 24, 64-67; Savino et al., 1985, Phytopathologia Mediterranea 24, 68-72; Goheen, 1988). At least five different phloem-restricted viruses belonging in two genera are involved in the aetiology of RW; i) four *Vitivirus* species, *Grapevine virus A* (GVA), *Grapevine virus B* (GVB), Grapevine virus C (GVC), and *Grapevine virus D* (GVD), (Martelli et al., 1997, Arch. Virol. 142, 1929-1932) and ii) one *Foveavirus,* named *Rupestris stem pitting associated virus-1* (RSPaV-1) (Meng et al., 1998, J. Gen. Virol. 79, 2059-2069) or *Grapevine rupestris stem pitting associated virus* (GRSPaV) (Zhang et al., 1998, Phytophathology 88, 1231-1237), a member of the recently established *Foveavirus* genus (Martelli and Jelkmann, 1998, Arch. Virol. 143,1245-1249). **The newly established genera *Vitivirus* and *Foveavirus* are phylogenetically related and together with the genera *Trichovirus, Capillovirus* and *Carlavirus* belong to the tentative family *Carlaviridae* (Hataya et al., 2000, Arch. Virol.145: 2503-2524).**

So far, respective detection of these viruses suffers from reduced reliability. Detection of grapevine RW and leafroll complex is based on bioassays, ELISA, and PCR. Bioassays are widely used but, they are time-consuming, demanding glasshouse infrastructure, and they are not reliable. The low concentration of RW and leafroll-related viruses and their uneven distribution in infected tissues along with their seasonal titer variation, make detection by ELISA methods hard to use, (requiring many samples from each plant), and unreliable due their low sensitivity. Moreover, vitiviruses are poor immunogens, yielding low titer antisera not suitable for use in ELISA. In addition, antisera raised against recombinant RSPaV-1 coat protein, are not yet suitable for ELISA (Minafra, 2000, In: Extended abstracts 13th Meeting ICVG, Adelaide, Australia, 12-17 March, p. 138-140). Detecting grapevine viruses even with present PCR methods available in the art remains difficult. Specific detection of RSPaV-1 variants or Vitiviruses is based on costly and time consuming PCR protocols with problems of detection polyvalence or low sensitivity and specificity. Furthermore the possible existence of unknown *Vitivirus, Foveavirus* and *Closterovirus* species can lead to inefficiency of diagnosis. Different PCR protocols have been proposed for the detection of GVA, GVB, GVC, and RSPaV-1, but the reported sequence variability for RSPaV-1 and GVB, has been a problem in designing primers with detection polyvalence. Increased variability of RSPaV-1 was observed in the sequence of several cDNA clones (Meng et al, Eur. J. Plant Pathology 105, 191-199; Soares et al., 2000, In: Extended abstracts 13th Meeting ICVG, Adelaide, Australia, 12-17 March, (p 41-42)). Phylogenetic analysis has revealed the existence of three major groups of sequence variants (Rowhani et al., 2000b, In: Extended abstracts 13th Meeting ICVG, Adelaide, Australia, 12-17 March, (p 41-42)). Similarly, extensive sequence variation exists within different GVB isolates (Shi et al., 2000, In: Extended abstracts 13th Meeting ICVG, Adelaide, Australia, 12-17 March, (p 48-49)) and two serologically distinct groups of GVA are reported, indicating that the selection of primers for the detection of GVA needs careful investigations (De Meyer et al., 2000, In: Extended abstracts 13th Meeting ICVG, Adelaide, Australia, 12-17 March, (p 30-34)). The use of two primer pairs in two different PCR assays has been proposed for the detection of RSPaV-1 with high polyvalence, although they do not detect all 'sequence variants' or isolates (Nolasco *et al.,* 2000, Eur. J. Plant Pathology 106, 311-318). Highly degenerate primers have been designed that can be used to amplify part of the HSP-70 homologue of closteroviruses (Karasev et al 1994, J. Gen. Virol. 75: 1415-1422). Two additional highly degenerate primers (HSP-P-1 and HSP-P-2) designed from the conserved phosphate 1 and 2 motifs of the *Closterovirus* HSP-70 homologues (Tian et al., 1996, Phytopathology 86: 1167-1172) were able to detect additional *Closterovirus* species. These primers along with group-specific, degenerate primers designed in the RdRp region of ORF 1 from vitiviruses, were used in two separate RT-PCR assays for the amplification of closteroviruses and vitiviruses infecting grapevine (Saldarelli et al., 1998, Eur. J. Plant Pathology 104, 945-950). Although these generic PCR detection methods known in the art for the detection of grapevine viruses allow the simultaneous detection of viruses belonging to one genus, problems such as low specificity and sensitivity, use of time consuming techniques for sample preparation (total-RNA or ds-RNA extraction), and PCR product detection by polyacrilamide gel electrophoresis and silver staining, make these assays unreliable for diagnosis and obstruct their large scale application. Template isolation is a critical step for routine use of PCR techniques. High concentration of phenolic compounds and polysaccharides that exist in grapevine inhibit *Taq* polymerase activity, and thus preventing virus detection. To overcome these problems, time consuming and complicated extraction procedures have been developed which can lead to sample contamination, and consequently in false-positives (Nassuth et al., 2000, J. Virol. Meth. 90, 37-49). Simplified and rapid sample processing procedures have been developed for virus detection in grapevine tissues, involving the direct spotting of plant sap on a nylon membrane, and processing of the membrane pieces by a thermal treatment before RT-PCR (La Notte, et al., 1997, J. Virol. Meth. 66, 103-108; Rowhani et al., 2000a, In: Extended abstracts 13th Meeting ICVG, Adelaide, Australia, 12-17 March, (p 148)), but problems exist because of high sample dilution that consequently decreases sensitivity.

Production of virus-free propagative material through certification schemes, is the only way for combating the great economic losses caused by viral diseases. This strategy needs efficient and reliable virus detection systems. The development of reliable, sensitive and simple methods for the detection of *Vitivirus, Foveavirus* and *Closterovirus* species is not only important for routine testing of propagative material in the field, but could also be useful in testing of explants produced by meristem tip culture. In addition multiplex detection of these viruses is important for the reduction of diagnostic costs.

Thus there is a need in the art for improved assays, of generic *Vitivirus, Foveavirus* and *Closterovirus* species detection in grapevine plants, being more efficient, reliable, less time consuming, with lower cost, having higher sensitivity, specificity and polyvalence, and enabling simultaneous detection of members belonging to said genera.

### SUMMARY OF THE INVENTION

The present invention provides for primers and optimised, highly sensitive, specific, reliable and inexpensive nested PCR-based detection methods, for the simultaneous generic detection of *Vitivirus, Foveavirus* and *Closterovirus* species in grapevine, involving an improved simple sample preparation suitable for large-scale applications.

Thus in one aspect, the invention comprises oligonucleotide primers useful for detection of members belonging to *Vitivirus, Foveavirus* and *Closterovirus* genus. These are oligonucleotides, capable of specifically binding to species of *Vitivirus* and *Foveavirus* genus, comprising any of the sequences listed as SEQ ID NO. 1, to NO. 5 and oligonucleotides, capable of specifically binding to species of *Closterovirus* genus, comprising any of the sequences listed as SEQ ID NO. 6, to NO. 12.

In a second aspect, the invention is directed to a nested reverse transcription-polymerase chain reaction (RT-PCR) method for co-detecting *Vitivirus, Foveavirus* and *Closterovirus* species in grapevine. Degenerate "inner" primers used for the nested PCR amplification of homologous sequence regions of viti- fovea- and closteroviruses are designed to have a Tm at least about 10°C higher than the "outer" oligonucleotide primers used in the first-stage RT-PCR. In addition the method comprises primers that are degenerated, contain deoxyinosine (dI), and are used along with respective homologous degenerated primers where dI is substituted by deoxyguanosine (dG) in regions of sequence homology.
The method also comprises:
(A) Isolating RNA from said sample suspected of being infected with one or more said viruses, or extracting RNA from said sample spotted in a nylon membrane.
(B) Performing a nested Reverse transcription-polymerase chain reaction to selectively amplify a targeted nucleotide sequence within said isolated RNA, said nested Reverse transcription-polymerase chain reaction comprising the steps of:
   (i) Amplifying using degenerate oligonucleotide primers in a first-stage reverse trancription polymerase chain reaction (RT-PCR) mixture, first targeted nucleotide sequences, said first targeted nucleotide sequences comprising: (a) a 363 base-pair region of the polymerase gene, from members of *Vitivirus* and *Foveavirus* genus, or (b) a 580-620 base-pair region of the heat shock protein 70 (HSP 70) homologue gene, from members of *Closterovirus* genus, or both (a) and (b).

   Where in said oligonucleotide primers specific for members of *Vitivirus* and *Foveavirus* genus comprise:
   Forward primer: 5'-WGCIAARGCIGGICARAC-3' (SEQ ID NO. 1),
   Reverse primers: 5'-RMYTCICCISWRAAICKCAT-3' (SEQ ID NO. 2), and
   5'-GCCGSWRAAGCKCAT-3' (SEQ ID NO. 3)

   Where in said oligonucleotide primers specific for members of *Closterovirus* genus comprise:
   Forward primers: 5'-GGIHTIGAITTYGGIACIACITT-3' (SEQ ID NO. 6), and
   5'-GTTYGGGACGACGTT-3' (SEQ ID NO. 7)
   Reverse primer: 5'-GTICCICCICCNAARTC-3' (SEQ ID NO. 8)
   and
   (ii) Removing an aliquot of the first-stage reaction mixture and amplifying, using degenerate inner oligonucleotide primers in a second-stage polymerase chain reaction mixture, second targeted nucleotide sequences internal to the first targeted nucleotide sequences, said second targeted nucleotide sequences comprising (a) a 201 base-pair region of the polymerase gene, from members of *Vitivirus* and *Foveavirus* genus, or (b) a 500-535 base-pair region of the HSP 70 homologue gene, from members of *Closterovirus* genus.
   Where in said inner oligonucleotide primers specific for members of *Vitivirus* and *Foveavirus* genus comprise:
   Forward primer: 5'-GGGGCARACIHTIGCITGYTT-3' (SEQ ID NO. 4)
   Reverse primer: 5'-AAIGCYTCRTARTCIGAITCNGT-3' (SEQ ID NO. 5)
   Where in said inner oligonucleotide primers specific for members of *Closterovirus* genus comprise:
   Forward primers: 5'-TTYGGGACGACGTTYAGYAC-3' (SEQ ID NO. 11) and
   5'-TYGGGACGACGTTYTCNAC-3' (SEQ ID NO. 12).
   Reverse primers: 5'-SCIGCIGMISWIGGYTCRTT-3' (SEQ ID NO. 9) and
   5'- GCGGMGSWGGG(dP)TCRTT-3' (SEQ ID NO. 10)
(C) Analysing said first and second-stage polymerase chain reaction mixtures following amplification to detect the presence or absence of said first and second targeted nucleotide sequences wherein the presence of the first and second targeted nucleotide sequences indicates the presence of said viruses in said sample suspected of being infected with said viruses.

In a third aspect, the invention is directed to a kit for co-detecting members of *Vitivirus, Foveavirus* and *Closterovirus* genus in a sample. Said kit comprising oligonucleotide primers specific for *Vitivirus* and *Foveavirus* members comprising the sequences listed as SEQ ID NO. 1, to NO. 5 and oligonucleotide primers specific for *Closterovirus* members comprising any of the sequences listed as SEQ ID NO. 6, to NO. 12

In another aspect, the invention is directed to a kit for detecting members of *Vitivirus,* and *Foveavirus* genus in a sample. Said kit comprising oligonucleotide primers specific for *Vitivirus* and *Foveavirus* members comprising the sequences listed as SEQ ID NO. 1, to NO. 5.

In yet another aspect, the invention is directed to a kit for detecting members of *Closterovirus* genus in a sample, said kit comprising oligonucleotide primers specific for *Closterovirus* members comprising any of the sequences listed as SEQ ID NO. 6, to NO. 12

### BRIEF DESCRIPTION OF THE DRAWINGS

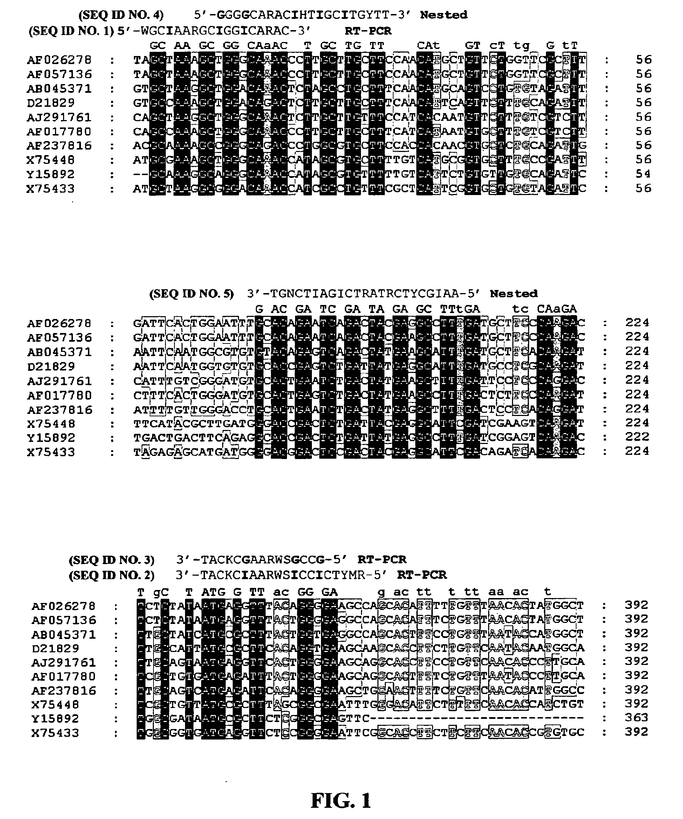

Figure 1. Depict a comparison of part of the polymerase gene sequences from RSPaV-1, *Apple stem pitting virus* (ASPV), *Cherry necrotic rusty mottle virus* (CNRMV), *Cherry green ring mottle virus* (CGRMV), GVA, GVD, and GVB, showing conserved nucleotide residues. Primers (SEQ ID NO. 1 to NO. 5) provided in the present invention for the detection of viti- and foveaviruses are indicated.

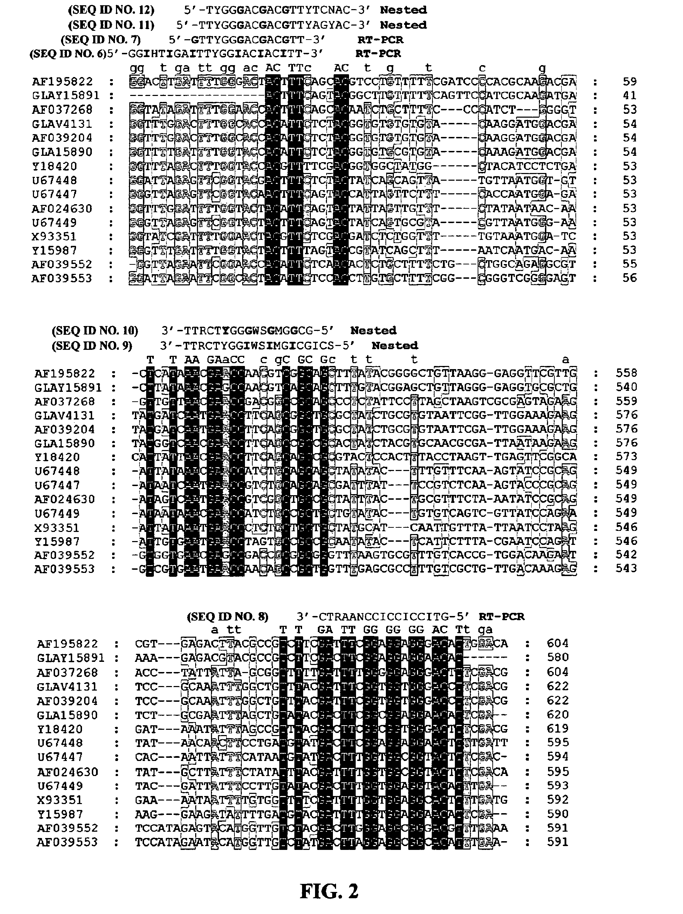

Figure 2. Comparison of part of the HSP 70 gene sequences from GLRaV-1, GLRaV-2, GLRaV-3, GLRaV-4, GLRaV-5, GLRaV-7, *Citrus tristeza virus* (CTV), *Little cherry virus* (LChV), *Beet pseudoyellows virus* (BPYV), *Cucurbit yellow stunting disorder virus* (CYSDV), *Tomato infectious chlorosis virus* (TICV), and *Tomato chlorosis virus* (ToCV), showing conserved nucleotide residues. Primers (SEQ ID NO. 6 to NO. 12) provided in the present invention for the detection of closteroviruses are indicated.

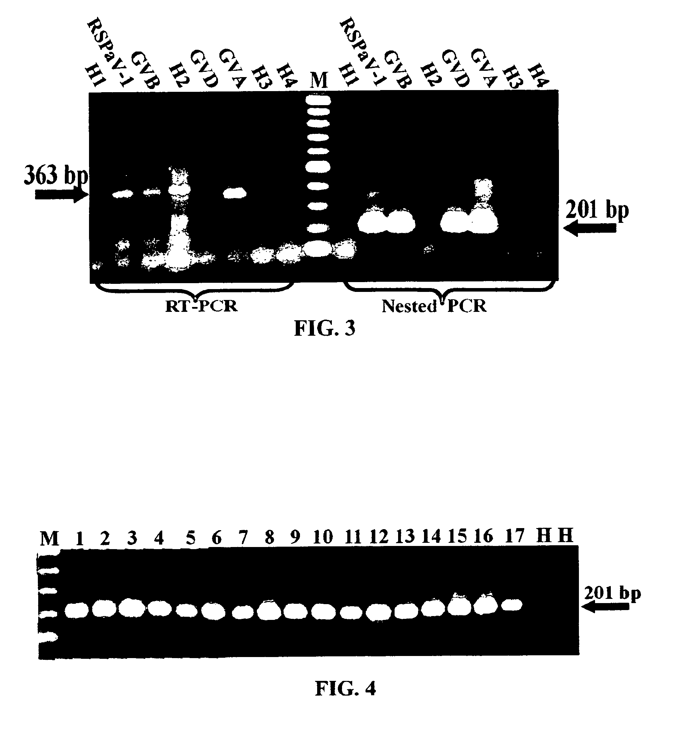

Figure 3. Agarose gel electrophoretic analysis of first one-step RT-PCR, and subsequent nested PCR products, obtained using primers (SEQ ID NO. 1, SEQ ID NO. 2, and SEQ ID NO. 3) and inner primers (SEQ ID NO. 4 and SEQ ID NO. 5) respectively, from total RNA preparations of grapevine leaf petioles infected with RSPaV-1, *N*. *benthamiana* with GVA, *N. occidentalis* with GVB, and *N. occidentalis* with GVD. (H=healthy controls. H1 and H2: grapevine, H3: *N*. *benthamiana,* H4: *N. occidentalis* M: 100 bp DNA ladder). Resolving of products from first RT-PCR amplification showed a non-specific amplicon, from healthy control H2, with molecular weight slightly higher than the expected. Additionally, faint or no amplification products were observed from some positive samples. Subsequent nested PCR was highly specific and sensitive, producing intensive bands in the case of positive samples without any non-specific products in the case of healthy controls.
Figure 4. Agarose gel electrophoretic analysis of nested PCR products, obtained from different grapevine plants infected with RSPaV-1 (lines 1-17), using spotted petiole extracts and primers (SEQ ID NO. 1, SEQ ID NO. 2, and SEQ ID NO. 3) and (SEQ ID NO. 4, SEQ ID NO. 5). (H: healthy grapevine, M: 100 bp DNA ladder).

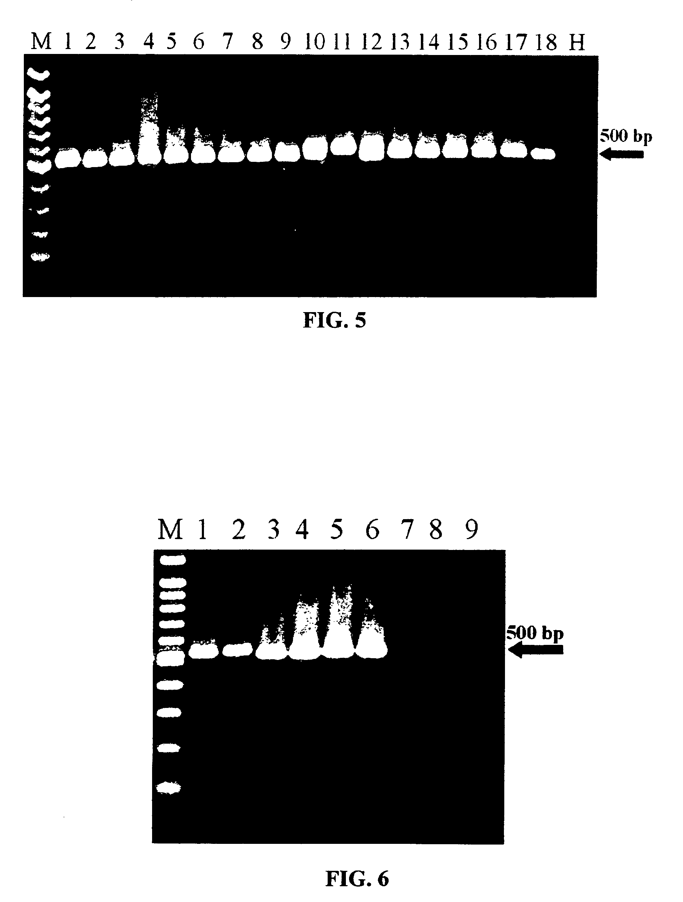

Figure 5. Agarose gel electrophoretic analysis of nested PCR products obtained from different GLRaV-infected grapevines (lines 1-18), using spotted petiole extracts, primers (SEQ ID NO. 6, SEQ ID NO. 7, and SEQ ID NO. 8) and inner primers (SEQ ID NO. 9, SEQ ID NO. 10, SEQ ID NO. 11, and SEQ ID NO. 12). GLRaV-7: lines 1-3; GLRaV-6: lines 4-6; GLRaV-5: lines 7-9; GLRaV-2: line 11; GLRaV-2 + GLRaV-1: lines 10 and 12; GLRaV-3: lines 13-15; and GLRaV-1: lines 16-18. H: healthy grapevine; M: 100 bp DNA ladder.
Figure 6. Agarose gel electrophoretic analysis of nested PCR products obtained from samples infected by different closteroviruses using spotted petiole extracts and primers (SEQ ID NO. 6, SEQ ID NO. 7, and SEQ ID NO. 8) and inner primers (SEQ ID NO. 9, SEQ ID NO. 10, SEQ ID NO. 11, and SEQ ID NO. 12). Lines 1-6 indicate; *Little cherry virus-1* (LChV-1) from cherry tree, CTV from citrus, BPYV from cucumber, CYSDV from cucumber, TICV from tomato, and ToCV from tomato; lines 7-9 indicate healthy controls from citrus, cucumber and tomato respectively; M: 100 bp DNA ladder).

### DETAILED DESCRIPTION OF THE INVENTION

The present invention is directed to the detection of *Vitivirus, Foveavirus,* and *Closterovirus* species, in grapevine samples. In particular the invention provides oligonucleotide primers and optimised reverse transcription (RT) polymerase chain reaction (PCR) methods with high sensitivity, specificity and polyvalence that allow rapid and multiplex detection of members belonging to said genera.

Primers for use in the invention were designed on highly conserved sequence regions of *Vitivirus, Foveavirus,* and *Closterovirus* species. Sequence homologies shared by *Vitivirus* and *Foveavirus* genera allowed the selection of consensus sequences in the viral RNA polymerase gene and the design of degenerate universal primer pairs targeting sequences of *Vitivirus* and *Foveavirus* species. Sequence alignments were done with nucleotide sequences obtained from GenBank (Fig 1). In addition, using the sequence data of several species of closteroviruses, we investigated the similarity of the different HSP 70 genes, and designed degenerate universal primer pairs, taking full account of nucleotide similarity. Primers were chosen for their maximum residue conservation, and minimum codon degeneracy (Fig 2). A strategy was then devised for rapid detection of vitiviruses and foveaviruses together with closteroviruses, based on an initial 'one step' RT-PCR amplification, followed by nested PCR.

For the initial amplification (RT-PCR), one pair of degenerate primers, which can theoretically amplify first targeted nucleotide sequences of viti- and foveaviruses, and one pair of degenerate primers, which can theoretically amplify first targeted nucleotide sequences of closteroviruses, were designed. One criterion used for the design of both primer pairs was, their minimum melting temperature *(Tm)* being approximately 45°C.

In addition two pairs of "inner" degenerate primers were designed for use in nested PCR, one for the amplification of viti- and foveaviruses, and one for the amplification of closteroviruses. These "inner" primers are derived from nucleotide sequences within the first targeted nucleotide sequences and flank second, smaller targeted nucleotide sequences contained within the first targeted nucleotide sequences. One criterion used for the design of both primer pairs was annealing temperature being higher (Tm≥55°C) than primers used for the initial amplification (RT-PCR).

Use of highly degenerate primers can be problematic giving low amplicon yield since only a small fraction of the oligonucleotide mixture is priming effectively. In the present invention, the primers designed for grapevine virus detection were modified by introducing deoxyinosine (dI) at degenerate positions for reducing their degeneracy without decreasing their potential target sequences. Comparison of results with those obtained using corresponding degenerate non-substituted primers showed that the use of dI-substituted primers can be advantageous in terms of both specificity and yield of the amplicons. The performance of corresponding degenerate primers without dI substitution was very poor showing a dramatic decrease in detection sensitivity of all nested PCR formats used. This primer design strategy allowing for reduced degeneracy of the primers and similar annealing temperature permitted the co-amplification of closteroviruses along with viti- and foveaviruses in the same initial RT-PCR reaction. The minimum Tm of primers containing deoxyinosines was calculated by adding 2°C for each A and T bases and degenerate positions except positions containing G or C, 4°C for each G and C bases and degenerate positions containing G or C, and 0°C for dI.

Although dl does have an additional base pair capability, it is not a neutral deoxynucleoside substitution in a PCR primer. Deoxyinosine base pairs with deoxycytidine (dC), but with lower affinity than deoxyguanosine (dG) with dC. The stability of dI base pairing with dC is higher than with deoxyadenosine (dA). In addition the stability of dI base pairing with deoxythymidine (dT) or dG is very weak (Wetmur and Sninsky, 1995, Nucleic acid hybridization and unconventional bases. In: M.A. Innis, D.H. Gelfand, J.J. Sninsky (Eds), PCR strategies, Academic Press Inc., San Diego, California, pp. 69-83.). In the present invention, in order to avoid annealing instability of the primers due to the incorporation of many deoxyinosines, substitutions were made only to fourfold degenerate positions.

Subsequently, the present invention, provides primers specific for *Vitivirus* and *Foveavirus* members comprising:
i. For use in RT-PCR
   Forward primer: 5'-WGCIAARGCIGGICARAC-3' (SEQ ID NO. 1)
   Reverse primer: 5'-RMYTCICCISWRAAICKCAT-3' (SEQ ID NO. 2)
ii. For use in nested PCR:
   Forward primer: 5'-GGGGCARACIHTIGCITGYTT-3' (SEQ ID NO. 4)
   Reverse primer: 5'-AAIGCYTCRTARTCIGAITCNGT-3' (SEQ ID NO.5)

In addition the present invention, provides primers specific for *Closterovirus* members comprising:
i. For use in RT-PCR
   Forward primer: 5'-GGIHTIGAITTYGGIACIACITT-3' (SEQ ID NO. 6)
   Reverse primer: 5'-GTICCICCICCIAARTCRWA-3' (SEQ ID NO. 8)
ii. For use in nested PCR
   Forward primer: 5'-TTYGGGACGACGTTYAGYAC-3' (SEQ ID NO. 11) and
   5'-TYGGGACGACGTTYTCNAC-3' (SEQ ID NO. 12)
   Reverse primers: 5'-SCIGCIGMISWIGGYTCRTT-3' (SEQ ID NO. 9)

In a second embodiment of the present invention, said primers are used in a nested RT-PCR method to detect the presence of *Vitivirus, Foveavirus,* and *Closterovirus* species, in grapevine samples.

Use of degenerate primers with high potential target sequences can be problematic due to increased possibility of nonspecific primer annealing. Nested RT-PCR methods show increased specificity and sensitivity over methods based on a unique PCR. Nested PCR confirms the specificity of the amplicon produced by the first amplification. In the present invention degenerate primers designed to anneal into the amplification product from the first reaction, improved dramatically the specificity and sensitivity of viti- and foveavirus detection solving problems of non-target sequence amplification and low yield of amplicons (Fig. 3).

To eliminate the possibility of contamination, it is vital to minimise the pipeting steps during the detection procedure. Most nested PCR formats reported use 1000 fold diluted product from the first PCR reaction to avoid activity of the outer primer pair from the first reaction. To avoid this step and to optimise the performance of the nested PCR reaction, the carryover of outer primers into the nested stage was minimised (only 5% of the first stage product it is added to the nested reaction). In addition the inner primers were designed to have a higher *Tm,* so higher annealing temperature is used in the nested reaction, in order to prevent the annealing of the outer primers.

According to sequence data and previous publications (Rossolini et al, 1994, Mol. Cell. Probes 8, 91-98), during PCR amplification dI appears to be preferentially recognised as dG by DNA polymerases, and for this reason in the PCR products dC is incorporated opposite to dI. In the present invention we discovered that a dI-containing primer can be used along with another respective homologous primer where dI is substituted by dG in the region of sequence homology, and this said homologous primer anneals more efficiently to the corresponding region of the denatured PCR products. In addition the length of said homologous primer could be lowered so that its Tm is similar to the Tm of the dI-containing primer and by this way its degeneracy could be also lowered. For example primer SEQ ID NO. 6 (5'-GGIHTIGA**I**TTYGG**I**AC**I**AC**I**TT-3') that contains 6 deoxyinosines and its degeneracy is 6, is used with along with homologous primer SEQ ID NO. 7 (5'-**G**TTYGG**G**ACGAC**G**TT-3') that has a similar Tm and its degeneracy is 2 (Fig. 2). Similarly primer SEQ ID NO. 2 (5'-RMYTC**I**CC**I**SWRAA**I**CKCAT-3') that contains 3 deoxyinosines and its degeneracy is 128, is used with along with primer SEQ ID NO. 3 (5'-**G**CC**G**SWRAA**G**CKCAT -3') that has a similar Tm and its degeneracy is 16 (Fig. 1). Finally primer SEQ ID NO. 9 (5'-SC**I**GC**I**GM**I**SW**I**GGYTCRTT-3') that contains 4 deoxyinosines and its degeneracy is 64, is used with along with primer SEQ ID NO. 10 (5'-GC**G**GM**G**SW**G**GG(dP)TCRTT-3') that has a similar Tm and its degeneracy is 16 (Fig. 2), the degeneracy of this primer was additionally lowered due to the incorporation of dP-CE Phosphoramidite (dP) instead of Y. Similarly, "inner" primers SEQ ID NO. 11 and NO. 12 having homologue region in their nucleotide sequences with the "outer" primer SEQ ID NO. 6 (Fig. 2), and "inner" primer SEQ ID NO. 4 having homologue region with the "outer" primer SEQ ID NO. 1 (Fig. 1), they contain dG instead of dI in said homologue regions. Said degenerate primers were dI is substituted by dG have higher annealing and amplification efficiency. With this strategy, the PCR amplification is much more efficient and primers that have many degenerate positions and high degeneracy can be used effectively in cases were high sensitivity of detection is required.

The nested RT-PCR method of the present invention comprises the following steps. RNA is first isolated from a tissue sample from grapevine plants, preferably cortical scrapings or petioles. Methods for isolation and preparation of RNA are available in the art. See, for example, Nassuth et al., (2000). Alternatively a simpler method that uses spotted samples can be applied. See, for example, La Notte et al., (1997) or even better a modification of this method (Dovas, unpublished) shown in example 1B. RNA extract is used in a first-stage RT-PCR, using "outer" oligonucleotide primers specific for the detection of vitiviruses and foveaviruses along with primers specific for the detection of closteroviruses, and the resulting mixture is subjected to an optimised RT-PCR thermocycling profile. At the end of the first-stage RT-PCR, an aliquot of the resulting mixture is carried over into a second-stage nested PCR. In this second-stage PCR reaction, the products of the first-stage RT-PCR reaction are combined with specific "inner" primers for the detection of closteroviruses, and in a separate PCR reaction with specific "inner" primers for the detection of vitiviruses and foveaviruses. These mixtures are subjected to thermocycling. The amplification products of the first- and second-stage polymerase chain reactions may be analysed to identify the presence or absence of the first and second targeted nucleotide sequences. Any one of several methods known in the art to detect amplified nucleotide sequences may accomplish identification of the amplification products. These methods include, but are not limited to, determination of size, subsequent cloning of amplification products, Southern blot hybridization, or DNA sequencing. The size of the product or products may be determined by electrophoresis through a gel, preferably an agarose gel, and stained with ethidium bromide.

The nested RT-PCR assay described in Examples 2 and 3 has been optimised to provide the greatest sensitivity, specificity and detection polyvalence. Optimisation involved modification of standard PCR protocol available in the art. (See particularly "Guide to Optimising PCR: (Perkin Elmer, 1994). Such modifications in both first-stage RT-PCR and nested PCR included altering the concentration of MgCl₂, primers, additives (DMSO and BSA), and enzymes in the PCR reaction mix, and adjusting experimental conditions, such as annealing temperature, and extension time.

Different annealing temperatures (Ta) in both the RT-PCR and the nested PCR were tested. The optimum Ta for nested PCR was 54°C. For RT-PCR, the optimum Ta was 43°C. Temperatures higher than 45°C were inefficient for primer annealing with some closteroviruses such as GLRaV-6 and GLRaV-5. By applying Ta lower than 43°C GLRaV-3 could not be constantly detected, possibly because at lower temperatures the secondary structure of the template may have interfered with priming. It is possible that dI substitutions very close to the 3'-terminus can cause annealing instability of the primers (Sommer and Tautz, 1989, Nucleic Acids Research, 16, 6749), especially when corresponding base opposite to dI in the template is dT or dG. Additional problems of effective template amplification of some *Closterovirus* isolates were observed in the RT-PCR assay when the annealing temperature was set to 43°C for 30 sec. Sequence data from one said isolate revealed that in the template, opposite to the dI located two bases from the 3'-terminus of the "outer" primer (SEQ ID NO. 6: 5'-GGIHTIGAITTYGGIACIAC**I**TT-3'), a dG existed. For this reason the annealing step of the first five cycles during the PCR amplification was modified by using a low non stringent annealing temperature of 38°C for 5 sec after the first annealing step (10 sec at 43°C). The relaxed annealing conditions allowed for the hybridisation of the primers (SEQ ID NO. 6) 3'-terminus and all isolates could be amplified (Example 2).

It was also found that addition of Dimethyl sulfoxide (DMSO) was necessary for effective amplification of GLRaV-3. Localised sequence regions possessing high melting temperatures have been reported to prevent the DNA amplification (McDowell et al, 1998, Nucleic Acids Res. 26, 3340-3347). When the thermal stability profile of the GLRaV-3 RT-PCR amplicon was calculated using a program (Steger, 1994, Nucleic Acids Res. **22**, 2760-2768), one region with localized high Tm (92°C) was observed. It is possible that if this localized sequence region possessing high Tm values is not denatured completely during the denaturation steps in RT-PCR, the DNA polymerase will be inhibited to read through these region and repression of the amplification will occur. Addition of 5% DMSO into the RT-PCR reaction permitted the successful detection of GLRaV-3 isolates. DMSO destabilizes inter- and intrastrand annealing and therefore disrupts base pairing, including secondary structure. In the case of GLRaV-3, DMSO may act by facilitating strand separation of the template DNA during denaturation and also destabilizing the intramolecular secondary structures within the template for efficient annealing of primers.

Based on these studies, the optimised thermal profiles for the RT-PCR and nested PCR and concentrations for constituents in their master mix are shown in examples 2 and 3.

The present invention provides for "kits" comprising the elements necessary to detect the presence or absence of *Vitivirus, Foveavirus* and *Closterovirus* species in a sample using the nested PCR method of the invention. Such a kit may comprise a carrier being compartmentalised to receive in close confinement therein one or more container means, such as tubes or vials. One of said container means may contain oligonucleotide primers capable of specifically binding to species of *Vitivirus* and *Foveavirus* genus comprising any of the sequences listed as SEQ ID NO. 1, to NO. 5 and oligonucleotides capable of specifically binding to species of *Closterovirus* genus comprising any of the sequences listed as SEQ ID NO. 6, to NO. 12, for use in a first-stage RT-PCR, and in a second-stage nested PCR. One or more said container means of such a kit may contain one or more enzymes or reagents along with any additional materials needed to carry out the detection method of the invention, such as buffers, extraction and purification reagents, nucleic acids, and dNTPs.

Sample preparation of grapevine samples for PCR testing is difficult, due to the effect of putative PCR-inhibitors. Total nucleic acid extraction frequently used, is costly, time consuming, increases the possibilities of contamination, and therefore not practical for routine diagnosis and analysis of several samples. The high sensitivity of this nested PCR method permitted the use of a simple spot sample preparation method by compensating the potential decrease in sensitivity due to the high dilutions required during spot sample preparation. Spotted samples combined with one-tube RT-PCR, allow simple, fast, and cost-effective analysis of a large number of samples. In the present invention, by using nested RT-PCR with degenerate dI-substituted primers, successful amplification with high specificity and sensitivity of sequences from three *Vitivirus* species (GVA, GVB, and GVD) was performed. In addition thirty different isolates of RSPaV-1 from field-grown infected V. vinifera plants, were successfully detected when spotted extracts were used as templates (selected results of RSPaV-1 detection in 17 samples are presented in Fig. 4). This nested RT-PCR method, using spotted extracts from RSPaV-1 infected grapevine petioles for template preparation, gave positive results with more than 1.000-fold dilution of the original extract. Successful detection of RSPaV-1 variants previously needed two different primer pairs in two different assays (Nolasco et al., 2000, Eur. J. Plant Pathology 106, 311-318). In addition, said nested RT-PCR was able to detect three more RSPaV-1 isolates that could not be detected using PCR and primer pairs described by Nolasco et al., (2000). Twenty different isolates of GLRaV-1 to -7, from single infected field-grown V. vinifera plants, were successfully detected when spotted extracts were used as templates (selected results of detection are presented in Fig. 5). Sequencing confirmed the respective viral origin of nested PCR amplicons. Specific identification of viruses detected is possible by sequencing of the nested PCR products, after cloning or directly by using primer SEQ ID NO. 10 (5'-GCGGMGSWGGG(dP)TCRTT-3') for closteroviruses and primer 5'-AAGGCYTCRTARTCGGAGTC-3' for fovea- and vitiviruses. Subsequently this developed nested PCR method allows rapid, reliable and inexpensive, simultaneous detection of all known species of Vitiviruses (GVA, GVB and GVD), Foveaviruses (RSPaV-1 variants) and Closteroviruses (GLRaV-1, GLRaV-2, GLRaV-3, GLRaV-4, GLRaV-5, GLRaV-6, and GLRaV-7), infecting grapevine (Fig. 3, Fig. 4, and Fig. 5). In addition other closteroviruses (LChV-1, CTV, BPYV, CYSDV, TICV, and ToCV), infecting plants such as cherry, citrus, cucumber, and tomato were also detected (Fig. 6). All these data strongly suggest that the nested RT-PCR described, is able to amplify additional viruses belonging to these genera that have not been identified yet, which is advantageous for achieving effective virus detection in certification schemes. This method can be useful in large-scale detection applications, because of the following advantages: 1) increased detection polyvalence, and multiplex generic detection of viruses that reduced time and cost per assay, by employing degenerated dI-containing primers 2) increased specificity and sensitivity, compared to other generic PCR methods known in the art for grapevine virus detection, by using a nested PCR format and by employing along with dI-containing primers additional homologous primers, wherein dI is substituted by dG. The increased sensitivity also allowed the use of a simple and rapid template preparation protocol, by spotting of plant sap extract on a nylon membrane, and a thermal treatment processing before RT-PCR. These improvements permit constant PCR amplification from petiole and cortical scraping preparations of infected grapevines, and enable multiplex detection of viruses belonging to three genera. The method of the present invention, compared to other generic PCR methods for grapevine virus detection known in the art has increased specificity and sensitivity, and compared to other PCR methods known in the art for specific single grapevine virus detection, has higher polyvalence and similar sensitivity and specificity.

### EXAMPLES

The following examples illustrate the present invention without limitation.

### 1. Sample preparation:

Samples can be prepared either by total RNA extraction (A), or by using a simple spot extraction method (B).
(A) Total RNA was extracted, from infected leaves according to a method described previously (Nassuth et al., 2000, J. Virol. Meth. 90, 37-49). Approximately 0.5 g tissue was homogenized with 5 ml lysis buffer (4 M guanidinium isothiocyanate, 0.2 M sodium acetate, pH 5.0, 25 mM EDTA, and 6% PVP-40 and 1% 2-mercaptoethanol). A 1-ml aliquot of the lysate was mixed with 67 µl 30% sarkosine and incubated for 10 min at 70°C. Approximately 600 µl of the mixture was applied to a Qiashredder spin column (Qiagen) and centrifuged for 2 min at 12.000 rpm. Next, 250 µl of the mixture was transferred to a new tube, mixed with 225 µl of 95% ethanol, applied onto an Rneasy column (Qiagen), and centrifuged for 45 s at 8000 g. The column was washed with 700 µl RW1 (Qiagen) and 500 µl RPE (Qiagen) (loaded and washed through by centrifugation for 15 s at 8000 g). An additional 500 µl RPE was loaded on the column and washed through by centrifugation for 5 min at 14 000 g. Finally, the RNA was eluted from the column by applying 100 µl H₂O, waiting for 1 min and centrifuging for 60 s at 8000 g.
(B) Spotted samples were prepared using an extraction protocol reported previously (La Notte et al., 1997) that was modified to give better and consistent results, consisting of the following steps: Hybond N + membranes (Roche Diagnostics GmbH, Germany) were wetted with 50 mM NaOH solution with 2.5 mM EDTA and then allowed to dry. A small piece (5 x 5 mm) of membrane was placed in the bottom of a sterile 1.5 ml microtube using a pincer. A small piece (ca. 0.1 g) of the petiole from middle-aged grapevine leaves was cut with a sterile blade and macerated in extraction buffer (0.5 M Tris, pH 8.2; 0,14 M NaCl; 10% PVP-40; 1% bovine serum albumin (BSA); 0.05% Tween 20 and 0,7% Na₂SO₃) at a 1:20 dilution. A small aliquot of this extract was centrifuged in 1.5 ml microtubes at 5000 g for two minutes, and 1 µl was carefully spotted onto the membrane into the microtube. Spotted membranes were allowed to dry either at room temperature or into a laminar flow before use or storage at -20° C. Release of viral template from the membranes was done by adding 200 µl of a "virus releasing medium", using a pipette tip with a wide hole. The microtubes (membrane and buffer) were incubated for 4 min at 94°C, vortexed, placed on ice for two minutes and then centrifuged at 12.000 rpm for one minute. Finally, 1 µl of the aqueous phase was added to 24 µl of RT-PCR reaction mixture. The "virus releasing medium" was made with hydrated insoluble PVPP (Sigma A-8022), by adding an equal volume of GES buffer (0.1M glycine-NaOH, pH 9.0, 50mM NaCl, 1mM EDTA) containing 0.5% Triton X-100, 1% b-mercaptoethanol, 2,8 M betaine (Sigma B-2754), and 0.5 µM of each primer "SEQ ID NO. 8" for closterovirus, and "SEQ ID NO. 2" for viti-and foveavirus detection. PVPP was previously hydrated by soaking 8 gr for at least two hours in 250 ml of GES buffer, and excess solution was discarded by decantation. After preparation, the virus-releasing medium can be divided in small aliquots (2-4 ml) and stored at -20°C until use.

### 2. RT-PCR amplification:

A 25-µl-reaction volume was used, containing 1 µl of either spotted plant extract or total RNA. The reaction mixture contained: 10 mM Tris-HCl [pH 8.8], 50 mM KCl, 1.5 mM MgCl₂, 0.1 % Triton X-100, 0.25 mM of each deoxyribonucleoside triphosphate (dNTP), 5.0 mM DTT, 5% DMSO, 5µg purified BSA (New England Biolabs, Inc., USA), 12 units RNASEOUT (Life Technologies™ MD, USA), 0.8 units Superscript™ II Rnase H Reverse Transcriptase (Life Technologies™ , MD, USA), 0,6 units Avian Myeloblastosis Virus Reverse Transcriptase (Finnzymes, Finland), 0.8 units Dynazyme™ II DNA Polymerase (Finnzymes, Finland), viti- and foveavirus specific primers (0.8 µM SEQ ID NO. 1; 1 µM SEQ ID NO. 2; and 0.8 µM SEQ ID NO. 3) and closterovirus specific primers (0.5 µM SEQ ID NO. 6; 0.2 µM SEQ ID NO. 7; and 1 µM SEQ ID NO. 8). The cycling profile was as follows: first step at 42°C for 50 min, second step at 50°C for 1 min, third step at 94°C for 4 min; five cycles segmented in step (a): 30 sec at 95°C, step (b): 10 sec at 43°C, step (c): 5 sec at 38°C, step (d): 15 sec at 72°C; thirty five cycles segmented in step (a): 30 sec at 95°C, step (b): 30 sec at 43°C, step (c): 20 sec at 72°C, followed by a final extension step at 72°C for 2 min. Amplifications were carried out in a PTC-200 peltier thermal Cycler (MJ Research), utilising thin-walled reaction tubes.

### 3. Nested PCR amplification:

Twenty µl nested PCR reactions were performed using 1 µl directly from the first RT-PCR product. The reaction mixture contained 10 mM Tris-HCl [pH 8.8], 50 mM KCl, 1.5 mM MgCl₂, 0.1 % Triton X-100, 0.2 mM of each dNTP, 1µg purified BSA, and 0.5 units Dynazyme™ II DNA Polymerase. For the detection of viti- and foveaviruses the following inner primers (1 µM SEQ ID NO. 4 and 1.5 µM SEQ ID NO. 5) were used. The cycling profile consisted of a first denaturising step at 95°C for 3 min, one step at 48°C for 15 sec, one step at 72°C for 15 sec and 39 cycles segmented in 20 sec at 95°C, 30 sec at 54°C, and 10 sec at 72°C (+ 1 sec after each cycle), followed by one final extension step at 72°C, for 2 min.

For the detection of closteroviruses the same buffer composition and cycling profile were used along with the following inner primers (1 µM SEQ ID NO. 9; 1 µM SEQ ID NO. 10; 0.5 µM SEQ ID NO. 11 and 1 µM SEQ ID NO. 12).

### 4. Detection of PCR products:

The reaction products were analysed by electrophoresis in 1.8% agarose gels in TAE buffer, stained with ethidium bromide, and visualized under UV light (Fig. 4, Fig. 5)

Many variations of the present invention are expected to occur to those skilled in the art in light of the above detailed description.

### SEQUENCE LISTING

<110> VITRO HELLAS
   Dovas, Crisostomos
<120> Highly sensitive nested PCR detection scheme for efficient multiplex detection of members of Vitivirus, Foveavirus and Closterovirus genus
   <130> VEUP1
<160> 12
   <170> PatentIn version 3.1
<210> 1
   <211> 18
   <212> DNA
   <213> Artificial sequence
   <220>
   <223> PRIMER
   <220>
   <221> misc_feature
   <222> (1)..(18)
   <223> In the primer sequence submitted n=inosine
   <400> 1
   wgcnaargcn ggncarac 18
<210> 2
   <211> 20
   <212> DNA
   <213> Artificial sequence
   <220>
   <223> PRIMER
   <220>
   <221> misc_feature
   <222> (1)..(16)
   <223> In the primer sequence submitted n=inosine
   <400> 2
   rmytcnccns wraanckcat 20
<210> 3
   <211> 15
   <212> DNA
   <213> Artificial sequence
   <220>
   <223> PRIMER
   <400> 3
   gccgswraag ckcat 15
<210> 4
   <211> 21
   <212> DNA
   <213> Artificial sequence
   <220>
   <223> PRIMER
   <220>
   <221> misc_feature
   <222> (1)..(21)
   <223> In the primer sequence submitted n=inosine
   <400> 4
   ggggcaracn htngcntgyt t 21
<210> 5
   <211> 23
   <212> DNA
   <213> Artificial sequence
   <220>
   <223> PRIMER
   <220>
   <221> misc_feature
   <222> (1)..(19)
   <223> In the primer sequence submitted n=inosine
   <220>
   <221> misc_feature
   <222> (20)..(23)
   <223> In the primer sequence submitted n=a or t or c or g
   <400> 5
   aangcytcrt artcngantc ngt 23
<210> 6
   <211> 23
   <212> DNA
   <213> Artificial sequence
   <220> zu
   <223> PRIMER
   <220>
   <221> misc_feature
   <222> (1)..(23)
   <223> In the primer sequence submitted n=inosine
   <400> 6
   ggnhtngant tyggnacnac ntt 23
<210> 7
   <211> 15
   <212> DNA
   <213> Artificial sequence
   <220>
   <223> PRIMER
   <400> 7
   gttygggacg acgtt 15
<210> 8
   <211> 17
   <212> DNA
   <213> Artificial sequence
   <220>
   <223> PRIMER
   <220>
   <221> misc_feature
   <222> (1)..(10)
   <223> In the primer sequence submitted n=inosine
   <220>
   <221> misc_feature
   <222> (11)..(17)
   <223> In the primer sequence submitted n=a or t or c or g
   <400> 8
   gtnccnccnc cnaartc 17
<210> 9
   <211> 20
   <212> DNA
   <213> Artificial sequence
   <220>
   <223> PRIMER
   <220>
   <221> misc_feature
   <222> (1)..(20)
   <223> In the primer sequence submitted n=inosine
   <400> 9
   scngcngmns wnggytcrtt 20
<210> 10
   <211> 17
   <212> DNA
   <213> Artificial sequence
   <220>
   <223> PRIMER
   <220>
   <221> misc_feature
   <222> (1)..(17)
   <223> In the primer sequence submitted n=dP-CE Phosphoramidite
   <400> 10
   gcggmgswgg gntcrtt 17
<210> 11
   <211> 20
   <212> DNA
   <213> Artificial sequence
   <220>
   <223> PRIMER
   <400> 11
   ttygggacga cgttyagyac 20
<210> 12
   <211> 19
   <212> DNA
   <213> Artificial sequence
   <220>
   <223> PRIMER
   <220>
   <221> misc_feature
   <222> (1)..(19)
   <223> In the primer sequence submitted n=a or t or c or g
   <400> 12
   tygggacgac gttytcnac 19

## Claims

1. A pool of degenerate oligonucleotide primers for detecting viruses belonging to *Vitivirus, Foveavirus* and *Closterovirus* genera, **characterised in that** it is used in a nested RT-PCR method and includes at least two primers selected from the group consisting of primers identified by Seq.IDs 1-12.

2. A method for the generic detection of members of *Vitivirus, Foveavirus* and/or *Closterovirus* genus in a sample suspected of being infected with said viruses, said method comprising:
(A) Isolating RNA from said sample suspected of being infected with one or more said viruses, or extracting RNA from said sample spotted in a nylon membrane,
(B) Performing a nested Reverse transcription-polymerase chain reaction to selectively amplify a targeted nucleotide sequence within said isolated RNA, said nested Reverse transcription-polymerase chain reaction comprising the steps of:
(i) Amplifying using degenerate oligonucleotide primers in a first-stage reverse trancription polymerase chain reaction (RT-PCR) mixture, first targeted nucleotide sequences, said first targeted nucleotide sequences comprising: (a) a 363 base-pair region of the polymerase gene from members of *Vitivirus* and *Foveavirus* genus, or (b) a 580-620 base-pair region of the heat shock protein 70 (HSP 70) homologue gene from members of *Closterovirus* genus, or both (a) and (b),
wherein said oligonucleotide primers specific for members of *Vitivirus* and *Foveavirus* genus comprise:
Forward primer: 5'-WGCIAARGCIGGICARAC-3' (SEQ ID NO. 1),
Reverse primers: 5'-RMYTCICCISWRAAICKCAT-3' (SEQ ID NO. 2), and
5'-GCCGSWRAAGCKCAT-3' (SEQ ID NO. 3) and/or
wherein said oligonucleotide primers specific for members of *Closterovirus* genus comprise:
Forward primers: 5'-GGIHTIGAITTYGGIACIACITT-3' (SEQ ID NO. 6), and
5'-GTTYGGGACGACGTT-3' (SEQ ID NO. 7)
Reverse primer: 5'-GTICCICCICCNAARTC-3' (SEQ ID NO. 8),
and
(ii) Removing an aliquot of the first-stage reaction mixture and amplifying, using degenerate inner oligonucleotide primers in a second-stage polymerase chain reaction mixture, second targeted nucleotide sequences internal to the first targeted nucleotide sequences, said second targeted nucleotide sequences comprising (a) a 201 base-pair region of the polymerase gene from members of *Vitivirus* and *Foveavirus* genus, or (b) a 500-535 base-pair region of the HSP 70 homologue gene from members of *Closterovirus* genus, wherein in said second stage PCR, amplification is carried out separately for members of the *Vitivirus* and *Foveavirus* genera and separately for members of the *Closterovirus* genus,
wherein said inner oligonucleotide primers specific for members of *Vitivirus* and *Foveavirus* genus comprise:
Forward primer: 5'-GGGGCARACIHTIGCITGYTT-3' (SEQ ID NO. 4)
Reverse primer: 5'-AAIGCYTCRTARTCIGAITCNGT-3' (SEQ ID NO. 5), and/or
wherein said inner oligonucleotide primers specific for members of *Closterovirus* genus comprise:
Forward primers: 5'-TTYGGGACGACGTTYAGYAC-3' (SEQ ID NO. 11) and
5'-TYGGGACGACGTTYTCNAC-3' (SEQ ID NO. 12),
Reverse primers: 5'-SCIGCIGMISWIGGYTCRTT-3' (SEQ ID NO. 9) and
5'- GCGGMGSWGGG(dP)TCRTT-3' (SEQ ID NO. 10),
(C) Analysing said first and second-stage polymerase chain reaction mixtures following amplification to detect the presence or absence of said first and second targeted nucleotide sequences wherein the presence of the first and second targeted nucleotide sequences indicates the presence of i) members of the *Closterovirus* genus, or ii) members of the *Vitivirus* and *Foveavirus* genera, in said sample suspected of being infected with said viruses.

3. The method of claim 2, wherein said sample is tissue from a species of Vitis.

4. A kit for codetecting members of *Vitivirus, Foveavirus* and *Closterovirus* genus in a sample, said kit comprising:
(A) at least two oligonucleotide primers specific for *Vitivirus* and *Foveavirus* members selected from the sequences listed as SEQ ID NO. 1, to NO. 5, and
(B) at least two oligonucleotide primers specific for *Closterovirus* members selected from sequences listed as SEQ ID NO. 6 to NO. 12

5. A kit for detecting members of *Vitivirus,* and *Foveavirus* genus in a sample, said kit comprising:
at least two Oligonucleotide primers specific for *Vitivirus* and *Foveavirus* members selected from the sequences listed as SEQ ID NO. 1, to NO. 5

6. A kit for detecting members of *Closterovirus* genus in a sample, said kit comprising:
at least two Oligonucleotide primers specific for *Closterovirus* members selected from the sequences listed as SEQ ID NO. 6, to NO. 12

## Patentansprüche

1. Eine anzahl von degenerierten Oligonukleotid- "Primer" fuer den Nachweis von Viren die zu den Virusgruppen *Vitivirus, Foveavirus* und *Closterovirus* gehören, mit der Eigenschaft, daß die in einer "nested-PCR"- Methode (Verschachtelte Polymerasen-Kettenreaktion Methode) benutzt werden und die zumindest zwei Primer beinhalten, ausgewählt aus der Gruppe von Primern 5, identifizierbar durch Seq.IDs 1-12.

2. Eine Methode für den allgemeinen Nachweis von Mitgliedern der Virusgruppen *Vitivirus, Foveavirus* und/oder *Closterovirus* in proben, die verdächtig sind, mit den erwähnten Viren infiziert zu sein, welche Methode beinhaltet:
(A) RNA Isolation von den besagten Proben, infiziert mit einem oder mehr als einen von den erwähnten Viren, oder RNA Extraktion aus den erwähnten Proben aufgetragen auf Nylon Membranen.
(B) Durchführung einer "nested" (gestuften) Reversen Transkription-Polymerase Kettenreaktion, um selektiv die gezielte Nukleotidsequenz zu amplfizieren, die in der besagten isolierten RNA enthalten ist. Die genannte Reverse Transkription-Polymerase Ketten Reaktion umfasset die folgenden Schritte:
(i) In einem ersten schritt, amplifikation der gezielten Nukleotidsequenzen durch die Anwendung von degenerierten Oligonukleotid "Primer" in der Mischung einer Reversen Transkription-Polymerase Ketten Reaktion, in der die benannten Nukleotid Sequenzen folgendes umfassen: (a) eine Region mit einer Länge von 363 Basenpaaren, aus dem Gen fuer die virale Polymerase aus Mitgliedern der Virusgruppen *Vitivirus* und *Foveavirus,* oder (b) einer 580-620 Basenpaare langen Region aus dem viralen Gen für das Protein das homolog ist zu dem "heat shock" 70 (HSP 70) protein aus Mietgliedern der Virusgruppe *Closterovirus,* oder beide (a) und (b),
worin die genannten Oligonucleotid-"primer" für Mitgliedern der Virusgruppen *Vitivirus* und *Foveavirus* spezifisch sind und umfassen:
"Forward Primer": 5'-WGCIAARGCIGGICARAC-3' (SEQ ID NO. 1),
"Reverse Primers": 5'-RMYTCICCISWRAAICKCAT-3' (SEQ ID NO. 2), und
5'-GCCGSWRAAGCKCAT-3' (SEQ ID NO. 3), und/oder
worin die genannten Oligonucleotid-"Primer" für Mitgliedern der Gattung *Closterovirus* spezifisch sind und umfassen:
"Forward Primers": 5'-GGIHTIGAITTYGGIACIACITT-3' (SEQ ID NO. 6), und
5'-GTTYGGGACGACGTT-3' (SEQ ID NO. 7)
"Reverse Primer": 5'-GTICCICCICCNAARTC-3' (SEQ ID NO. 8),
und
(ii) Entfernung von einem Teil aus der Reaktions-Mischung des ersten Schrittes und Amplifikation unter Benutzung von degenerierten "inneren" Oligonukleotid Primer in einem zweiten Schritt, einer Polymerase-Kettenreaktions-Mischung, wobei die zweite Zielnukleotid Sequenz, sich innerhalb der ersten Zielnukleotid Sequenz befindet, wobei die genannte zweite Zielnukleotid Sequenz folgendes umfaßt: (a) eine 201 Basen Paare langen Region des viralen Polymerase Gens für Mitgliedern der Virusgruppen *Vitivirus* und *Foveavirus,* oder (b) eine 500-535 Basepaaren langen Region des viralen Gens homolog zu HSP 70, wobei, die besagte zweite Stufe (Schritt) der PCR Amplifikation, für die Mitgliedern der *Virusgruppen Vitivirus* und *Foveavirus,* getrennt ausgeführt wird, von derjenigen die für die Mitgliedern der Virusgruppe *Closterovirus*
worin, die genannten "inneren" Oligonukleotid Primer die spezifisch für Mitgliedern der Gattungen *Vitivirus* and *Foveavirus* sind, folgendes umfassen:
"Forward Primer": 5'-GGGGCARACIHTIGCITGYTT-3' (SEQ ID NO. 4)
"Reverse Primer": 5'-AAIGCYTCRTARTCIGAITCNGT-3' (SEQ ID NO. 5),
und/oder
worin, die genannten inneren Oligonukleotid-Primer die spezifisch für Mitgliedern der Virusgruppen *Closterovirus* sind, folgendes umfassen:
Forward Primers: 5'-TTYGGGACGACGTTYAGYAC-3' (SEQ ID NO. 11) und
5'-TYGGGACGACGTTYTCNAC-3' (SEQ ID NO. 12),
Reverse Primers: 5'-SCIGCIGMISWIGGYTCRTT-3' (SEQ ID NO. 9) und
5'- GCGGMGSWGGG(dP)TCRTT-3' (SEQ ID NO. 10),
(C) Analyse der genannten Zwei-Stufen Polymerasen Kettenreaktions-Mischungen, nach der Amplification, um die Anwesenheit oder Abwesenheit der genannten ersten und zweiter Zielnukleotid Sequenzen nachzuweisen, wobei die Anwesenheit von der ersten und der zweiten Zielnukleotid Sequenzen, die Anwesenheit von i) Mitgliedern der Virusgruppe *Closterovirus,* oder ii) Mitgliedern der Virusgruppen *Vitivirus* und *Foveavirus,* in den erwähnten Proben, verdächtigt mit den besagten Viren infiziert zu sein, andeuten.

3. Die Methode 2 worin die besagte Probe aus Gewebe einer Pflanze der Gattung *Vitis* herkommt.

4. Ein "kit" (Zusammenstellung von Reagenzien) für den gleichzeitigen Nachweis von Mitgliedern der Virusgruppen *Vitivirus, Foveavirus* und *Closterovirus* in einer Probe, wobei das erwähnte "kit" folgendes umfaßt:
(A) mindestens zwei Oligonucleotid Primer, spezifisch für Mitglieder der Virusgruppen *Vitivirus* and *Foveavirus,* welche Primer aus den Sequenzen erwähnt als SEQ ID NO. 1, bis NO. 5 ausgewählt sind, und
(B) mindestens zwei Oligonucleotid Primer, spezifisch für Mitgliedern der Virusgruppe *Closterovirus* welche Primer aus den Sequenzen erwähnt als SEQ ID NO. 6, bis NO. 12 ausgewählt sind.

5. Ein "kit" für den gleichzeitigen Nachweis von Mitgliedern der Virusgruppen *Vitivirus* und *Foveavirus* in einer Probe, wobei das erwähnte "kit" folgendes umfaßt:
mindestens zwei Oligonucleotid Primer, spezifisch für Mitgliedern der Virusgruppen *Vitivirus* and *Foveavirus,* welche Primer aus den Sequenzen erwähnt als SEQ ID NO. 1, bis NO. 5 ausgewählt sind.

6. Ein "kit" für den Nachweis von Mitgliedern der Virusgruppe *Closterovirus* in einer Probe, wobei das erwähnte "kit" folgendes umfaßt:
mindestens zwei Oligonucleotid Primer, spezifisch für Mitgliedern der Virusgruppe *Closterovirus* welche Primer aus den Sequenzen erwähnt als SEQ ID NO. 6, bis NO. 12 ausgewählt sind.

## Revendications

1. Un groupe d'amorces dégénérées oligonucléotidiques, pour la détection de genres *Vitivirus, Foveavirus* et *Closterovirus* **caractérisé par** son utilisation par la méthode de «nested RT-PCR» et comprenant au moins deux amorces sélectionnées du groupe qui contient des amorces identifiées par Seq.IDs 1-12.

2. Une méthode pour la détection générique des virus membres des genres *Vitivirus, Foveavirus* et/ou *Closterovirus* dans un échantillon suspecté d'être infecté par les virus dits et par la méthode dite, qui consiste de:
(A) L'isolement du ARN de l'échantillon suspecté d'être infecté par un ou plusieurs des virus dits, ou l'extraction du ARN de l'échantillon dit, déposé sur une membrane de nylon
(B) L'application de la méthode «nested reverse transcription-polymerase chain reaction» pour l'amplification sélective d'une séquence nucléotidique cible dans l'ARN isolé comme mentionné. La méthode consiste aux étapes suivantes :
(i) Amplification, en utilisant d'amorces dégénérées oligonucléo-tidiques dans une première étape d'une «reverse transcription-polymerase chain reaction» (RT-PCR), des séquences nucléotidiques cibles qui comprennent: (a) une région de 363 pairs de bases du gène de la polymérase pour les membres des genres *Vitivirus* et *Foveavirus* ou (b) une région de 580-620 pairs de bases du gène homologue à la protéine «heat shock» 70 (HSP 70) pour les membres du genre *Closterovirus,* ou les deux (a) et (b),
où les amorces oligonucléotidiques mentionnées, spécifiques des membres de genres *Vitivirus* et *Foveavirus,* comprennent:
Amorce en avant: 5'-WGCIAARGCIGGICARAC-3' (SEQ ID NO. 1),
Amorces en arrière: 5'-RMYTCICCISWRAMCKCAT-3' (SEQ ID NO. 2), et 5'-GCCGSWRAAGCKCAT-3' (SEQ ID NO. 3), et/ou
où les amorces oligonucléotidiques mentionnées spécifiques des membres du genre *Closterovirus* comprennent:
Amorces en avant: 5'-GGIHTIGAITTYGGIACIACITT-3' (SEQ ID NO. 6), et 5'-GTTYGGGACGACGTT-3' (SEQ ID NO. 7)
Amorce en arrière: 5'-GTICCICCICCNAARTC-3' (SEQ ID NO. 8),
et
(ii) Ecart d'un aliquote du mélange de la réaction de la première étape et amplification en utilisant des amorces dégénérées oligonucléotidiques internes dans une deuxième étape de «polymerase chain reaction», les secondes séquences nucléotidiques cibles, étant internes par rapport aux premières séquences nucléotidiques cibles, dits secondes séquences nucléotidiques cibles comprenant (a) une région de 201 pairs de bases du gène de la polymérase pour les membres des genres des *Vitivirus* et *Foveavirus,* ou (b) une région de 500-535 pairs de base du gène homologue à HSP 70 pour les membres du genre de *Closterovirus,* cette deuxième étape d'amplification par PCR s'effectuant séparément pour les membres des genres des *Vitivirus* et *Foveavirus,* et séparément pour les membres du genre de *Closterovirus,*
où les amorces oligonucléotidiques internes mentionnées, spécifiques des membres de genres *Vitivirus* et *Foveavirus,* comprennent:
Amorce en avant: 5'-GGGGCARACIHTIGCITGYTT-3' (SEQ ID NO. 4)
Amorce en arrière: 5'-AAIGCYTCRTARTCIGAITCNGT-3' (SEQ ID NO. 5), et/ou
où les amorces oligonucléotidiques internes mentionnées, spécifiques des membres du genre de *Closterovirus* comprennent:
Amorces en avant: 5'-TTYGGGACGACGTTYAGYAC-3' (SEQ ID NO.11) et 5'-TYGGGACGACGTTYTCNAC-3' (SEQ ID NO. 12),
Amorces en arrière: 5'-SCIGCIGMISWIGGYTCRTT-3' (SEQ ID NO. 9) et 5'- GCGGMGSWGGG(dP)TCRTT-3' (SEQ ID NO. 10),
(C) L'analyse des mélanges des réactions PCR de la première et deuxième étape mentionnées après amplification pour détecter la présence ou l'absence des premières et des deuxièmes séquences nucléotidiques cibles, où la présence des premières et des secondes séquences nucléotidiques cibles indique la présence de i) membres du genre de *Closterovirus,* ou ii) membres des genres de *Vitivirus* et *Foveavirus,* dans l'échantillon dit, suspecté d'être infecté par les virus dits.

3. La méthode de revendication 2, pour laquelle l'échantillon dit c'est du tissu d'une espèce du genre *Vitis.*

4. Un kit pour la détection simultanée de membres des genres de *Vitivirus, Foveavirus* et *Closterovirus* dans un échantillon, le kit dit se consistant de:
(A) au moins deux amorces oligonucléotidiques spécifiques des membres de genres *Vitivirus* et *Foveavirus,* sélectionnées parmi les séquences mentionnées dans SEQ ID NO. 1, à NO. 5, et
(B) au moins deux amorces oligonucléotidiques spécifiques des membres du genre de *Closterovirus,* sélectionnées parmi les séquences mentionnées dans SEQ ID NO. 6, à NO. 12.

5. Un kit pour la détection de membres des genres de *Vitivirus* et *Foveavirus* dans un échantillon, le kit dit se consistant de:
au moins deux amorces oligonucléotidiques spécifiques des membres de genres *Vitivirus* et *Foveavirus,* sélectionnées parmi les séquences mentionnées dans SEQ ID NO. 1, à NO. 5

6. Un kit pour la détection de membres des genres de *Closterovirus* dans un échantillon, le kit dit se consistant de:
au moins deux amorces oligonucléotidiques spécifiques des membres du genre de *Closterovirus,* sélectionnées parmi les séquences mentionnées dans SEQ ID NO. 6, à NO. 12.
